# EUROPÄISCHE PATENTANMELDUNG

(11) **EP 2 518 492 A1**
(43) Veröffentlichungstag der Anmeldung: **31.10.2012**
(21) Anmeldenummer: 12075043.5
(22) Anmeldetag: 27.04.2012
(51) Int. Cl.: G01N 33/46, G01N 21/64, B07C 5/14

(54) **Nicht-invasives Messverfahren zur Unterscheidung von Kernholz und Splintholz**

(30) Priorität: 30.04.2011 DE 102011100448
(71) Anmelder: LTB-Lasertechnik Berlin GmbH, 12489 Berlin (DE)
(72) Erfinder: Stankovic, Goran, 12249 Berlin (DE); Leupold, Dieter, 14612 Falkensee (DE); Scholz, Matthias, 15838 Mellensee (DE); Zscheile, Matthias, 83059 Kolbermoor (DE)

(57) **Zusammenfassung**

Für eine rationelle industrielle Holzverarbeitung ist eine schnelle Unterscheidung von dauerbeständigem Kernholz und witterungsanfälligem Splintholz erforderlich. Eingesetzt werden dazu auch Verfahren auf der Basis von Fluoreszenzspektren, wobei jedoch Kernholz und Splintholz durch Zuordnung von charakteristischen Fluoreszenzspektren unterschieden werden sollen. Bei dem erfindungsgemäßen Messverfahren werden für eine bekannte Holzart (HA) bei einer anregenden Wellenlänge λₐ an einem Messort Mᵢ erzeugte Fluoreszenzspektren Fᵢ von Kernholz (KH) und Splintholz (SH) miteinander verglichen und auf einen Schnittpunkt S hin untersucht. Existiert ein Schnittpunkt S, werden ober- und unterhalb der Wellenlänge λₐ des Schnittpunkts S Wellenlängen λ₁, λ₂ festgelegt und die zugehörigen Intensitäten lᵢ detektiert. Anschließend wird für Kernholz (KH) und Splintholz (SH) die Größenrelation der Intensitäten lᵢ ermittelt. Diese kann auch in Form eines Zahlenwerts Zᵢ ausgedrückt werden. Bei der holzindustriellen Verarbeitung müssen dann nur noch im Messort Mᵢ detektierte Intensitäten lᵢ bei den Wellenlängen λ₁, λ₂ ermittelt und der Zahlenwert Zᵢ bestimmt und verglichen werden, um eine zuverlässige und schnelle Zuordnung des Messorts Mᵢ zu Kernholz (KH) oder Splintholz (SH) zu erhalten.

## Beschreibung

Die Erfindung bezieht sich auf ein nicht-invasives Messverfahren zur Unterscheidung von Kernholz und Splintholz auf Basis von monochromatisch angeregten Fluoreszenzspektren und auf eine Anwendung des Messverfahrens.

Zahlreiche Holzarten verfügen über einen Stammaufbau aus zentralem Kernholz und peripherem Splintholz. Kernholz bezeichnet im Stammquerschnitt die physiologisch nicht mehr aktive, meist dunkle innere Zone, die sich deutlich vom äußeren, hellen Splintholz unterscheidet. Echtes Kernholz enthält überwiegend farbige Kernstoffe (Phenole), welche die Zellwände imprägnieren und die Dauerhaftigkeit des Holzes (Festigkeit, Resistenz gegen Feuchtigkeit und Schädlinge) erhöhen. Reifholz ist ebenfalls Kernholz, hebt sich aber farblich nicht vom Splintholz ab. Festes Kernholz wird bevorzugt als massives Nutzholz verarbeitet. Zu unterscheiden sind echte und falsche Kernholzbäume, deren Kernholz nicht die Dauerhaftigkeit von echtem Kernholz zeigt. Zu den echten Kernholzbäumen zählen Kiefer, Eiche und Ebenholz. Weiterhin werden Steineiben und Fichten mit einem hellen Kernholz zu den echten Kernholzbäumen gezählt. Zu den falschen Kernholzbäumen gehören Kirsche, Esche und Buche. Splintholz ist das junge, physiologisch aktive Holz unterhalb des Kambiums im Stamm eines Baumes. Mit zunehmendem Alter verliert das Splintholz an Lebenskraft und wandelt sich bei zahlreichen Baumarten in Kernholz (Verkernung). Splintholzbäume sind Bäume mit verzögerter Kernholzbildung, es bestehen keine Farb- und Feuchteunterschiede zwischen Kern- und Splintholz. Hierzu gehören z. B. Birke, Erle und Ahorn. Ihr Holz hat über den gesamten Querschnitt Splintcharakter. Splintholz weist als Nutzholz keine natürliche Dauerhaftigkeit auf. Für Holz im Außeneinsatz wird das Splintholz daher bei der Verarbeitung entweder entfernt (und z.B. bei Faserplatten verwendet) oder dessen Dauerhaftigkeit durch Einbringen chemischer Holzschutzmittel erhöht. Oft erfolgt auch ein Einsatz als Spanholz. Grundsätzlich kann im Außenbereich das robustere Kernholz und im Innenbereich das empfindlichere Splintholz eingesetzt werden.

Im Elementebau wird Holz in der Regel in Brett- oder Balkenform für den Türen-, Fenster- und Möbelbau eingesetzt. Durch die zunehmende Verknappung an Exotenhölzern gelangt immer mehr die Verwendung von nachhaltig verfügbaren einheimischen Holzarten in den Vordergrund. Dabei sollen diese jedoch möglichst ohne zusätzliche thermische oder chemische Modifikationsverfahren zum Einsatz gelangen. Qualitätsbestimmend ist daher die zuverlässige Bestimmung von speziellen Holzmerkmalen, zu denen insbesondere die Unterscheidung von Kernholz und Splintholz zählt. Die Zuverlässigkeit bezieht sich dabei auf eine Unterscheidbarkeit sowohl bei frisch eingeschnittenem oder sägefrischem als auch bei nach der Trocknung oberflächenbearbeitetem (z.B. gesägtem, gehobeltem oder geschliffenem) Holz. Bei der industriellen Verarbeitung von Massivholz, hauptsächlich bei Zuschnitt- und Sortierprozessen, kommen zunehmend automatische Verfahren zur Holzmerkmalsbestimmung zur Anwendung. Dabei handelt es sich hauptsächlich um multisensorielle Messsysteme zur in-situ Oberflächendetektion verschiedener Schnitthölzer in sägefrisch feuchtem sowie gebrauchsfertig getrocknetem Zustand in Form von Brettware, Kanteln, Lamellen, Friese, Schnittholz und Parkettriemen. Diese Scannersysteme umfassen optische Komponenten, wie beispielsweise mehrkanalige CMOS-Kameras oder hochauflösende Farbkameras, und Lasertriangulationssysteme zur hochauflösenden Dimensionsvermessung, optional können noch ein- oder mehrdimensional wirkende Röntgengeräte zur Bestimmung im Holz auftretender Dichteunterschiede hinzukommen. Nachteilig sind hier die hohen Kosten. Optische Systeme werden auch nach chemischen Vorbehandlungen der Oberfläche, beispielsweise Einfärbungen, angewendet. Auch akustische Messsysteme zur Ermittlung und Auswertung der holzmerkmals- und fehlerstellenabhängigen Eigenfrequenzänderungen der Prüflinge als Folge von deren Fremderregung gelangen zum Einsatz. Akustische und mikrowellenbasierte Verfahren unterliegen aber aufgrund des Feuchtigkeitseinflusses relativ hohen Ungenauigkeiten. Messverfahren, die auf der Auswertung von angeregten Fluoreszenzspektren beruhen, sind hingegen relativ kostengünstig und zuverlässig.

### Stand der Technik

In der US 5 220 172 A wird der Holzgehalt (Lignin) in einem Holzgemisch durch Messung der angeregten Fluoreszenz bestimmt. Es wird eine proportionale Abhängigkeit zwischen der Fluoreszenzintensität und dem Holzgehalt festgestellt.

Aus der US 6 757 354 B2 ist ein Messverfahren bekannt, mit dem Holz mit zwei elektromagnetischen Strahlquellen bestrahlt und die Fluoreszenz mit einer Photodiode detektiert wird. Bei den Lichtquellen handelt es sich aber bevorzugt um Röntgenstrahler, die in einem speziellen Winkelverhältnis zueinander angeordnet sind. Es wird die Differenz zwischen dem eingestrahlten und dem transmittierten Röntgenflux zur Detektion von Grenzen zwischen Hartholz und Splintholz bei einem Holzprüfling gemessen.

Auch in der Literatur wird vielfältig über die Anwendung von FluoreszenzSpektroskopie bei der Charakterisierung von Holz berichtet. In der Veröffentlichung **V1** (T. Werner, H. Pecina "Versuche zur Anwendung der FluoreszenzSpektroskopie in der Holztechnologie für die Bewertung von Pilzbefall in Holz" in Holz als Roh- und Werkstoff 53 (1995), p. 49-55) beispielsweise mit dem Ziel, Pilzbefall nachzuweisen (Bewertung von biologischen Abbauprozessen). Es wird ausgeführt, dass zur Untersuchbarkeit der Fluoreszenz von Proben geeignete Anregungswellenlängen gefunden werden müssen. Die Form der Fluoreszenzspektren von Stoffgemischen aus mehreren Komponenten ist immer von der Anregungswellenlänge abhängig. Es werden Fluoreszenzspektren von Kiefern-Kernholz und Kiefern-Splintholz bei 270 nm und 450 nm mit und ohne Pilzbefall dargestellt. Die gemessenen Fluoreszenzspektren werden durch Integralbildung zur Ermittlung der emittierten Gesamtenergie ausgewertet. Es konnten Integrationsgrenzen bei bestimmten Wellenlängen gefunden werden, zwischen denen ein Zusammenhang zwischen dem Wert des Integrals und der Schädigung des Holzes durch Pilzbefall aufgrund von Masseverlust besteht. Durch Relation der Fluoreszenzemission zwischen diesen Grenzen in Bezug auf die gesamte Fluoreszenzemission kann ein energetischer Anteil als Maß für die Schädigung definiert werden. Problematisch ist das Auffinden der speziellen Wellenlängen, da die gefundenen Zusammenhänge nur äußerst schwach sind und großen Streuungen unterliegen.

Aus der Veröffentlichung **V2** (V. Piuri, F. Scotti: "Design of an Automatic Wood Types Classification System by Using Fluorescence Spectra", in IEEE Trans. on Systems, Ma, and Cybernetics - Part C: Appl. and Rev., Vol.40, No.3, May 2010, p.358-366) ist die Auswertung von Fluoreszenzspektren zur Bestimmung von Holzarten bekannt. Es wird versucht, jeder Holzart ein spezielles Fluoreszenzspektrum eindeutig zuzuordnen. In Sortieranlagen sollen dann verschiedene Holzreste anhand ihres Fluoreszenzspektrums erkannt werden. Weiterhin ist den Veröffentlichungen **V3, V4** (K. Nkansah and B. Dawson-Andoh: "Rapid chacterization of biomass using fluorescence spectroscopy coupled with multivariate data analysis. I. Yellow poplar (Liriodendron tulipifera L.) in J. of Renewable and Sustainable Energy 2, 023103 (April 2010) und II. Northern red oak (*Quercus rubra*) in J. of Renewable and Sustainable Energy 2, 043101 (August 2010)) zu entnehmen, dass für beide Kernholzaumsorten (Tulpenbaum, Roteiche) Fluoreszenzspektroskopie angewendet wird, um einige physikalische und chemische Eigenschaften in Bezug auf eine Umwandlung in Bioenergie und Bioprodukte zu bestimmen. Für beide Holzsorten werden für das Hartholz Anregungs- und Fluoreszenzspektren aufgezeigt. Die Auswertung der Spektren erfolgt dann mit einer relativ aufwändigen multivarianten Datenanalyse zur Selektion von wesentlichen, unabhängigen Informationen aus der Fülle der Spektrendaten.

Der der Erfindung nächstliegende Stand der Technik wird in der Veröffentlichung **V5** (S.T. Sum et al. "Laser-excited fluorescence spectra of eastern SPF wood species. An optical technique for identification and separation of wood species?" in Wood Sci. Technol. 25, p. 405-413 (1991)) beschrieben. Es werden blockförmige Holzproben aus Kernholz und Splintholz jeweils aus Fichte (S - spruce), Kiefer (P - pine) und Tanne (F - fir) eingesetzt. Dabei werden diese so zur ringförmigen Maserung geschnitten, dass sich zwei radiale (quer zur Maserung) und zwei tangentiale (parallel zur Maserung) Oberflächen ergeben. Die Holzproben werden durch Einfrieren in frischem Zustand gehalten. Die Oberflächen werden mit gepulster monochromatischer Laserstrahlung der Wellenlänge 308 nm zur Fluoreszenz angeregt und vollständige Fluoreszenzspektren detektiert. Dabei werden einmal die von der radialen Oberfläche abgestrahlten Fluoreszenzspektren jeweils für Splintholz und Kernholz pro Holzsorte nebeneinander dargestellt. Analog werden die von der tangentialen Oberfläche abgestrahlten Fluoreszenzspektren jeweils für Splintholz und Kernholz pro Holzsorte nebeneinander dargestellt. Es zeigen sich Fluoreszenzspektren mit ausgeprägten Peaks. Die Auswertung bezieht sich auf die Peakwellenlänge und die Peakbreite (Spektrumbreite bei 75% Peakintensität). Es wird versucht, über diese Parameter eine zuverlässige Zuordnung zu Kernholz und Splintholz zu erhalten. Aufgrund von sich veränderndem Wassergehalt (durch Auftauen der gefrorenen Holzproben) und photochemischem Ausbleichen der Fluoreszenz (durch hohe Lasereinstrahlung, da immer ein komplettes Fluoreszenzspektrum erregt werden muss) während der Detektionen, ergeben sich aber keine zuverlässigen Parameterwerte bzw. Zuordnungen zu Kernholz und Splintholz. Es wird vermutet, dass bei anderen Anregungswellenlängen größere Peakunterschiede aufgefunden werden könnten, sodass das Messverfahren dann auch für eine schnelle Holzsortierung ertüchtigt werden könnte.

### Aufgabenstellung

Die **Aufgabe** für die vorliegende Erfindung ist darin zu sehen, ein Messverfahren zur Unterscheidung von Kernholz und Splintholz auf Basis von monochromatisch angeregten Fluoreszenzspektren anzugeben, das in einfacher und schneller sowie zuverlässiger und kostengünstiger Weise eine Unterscheidung von Kernholz und Splintholz ermöglicht. Insbesondere soll eine automatisierte in-situ Integration des Messverfahrens in Produktionsprozesse möglich sein. Die **Lösung** für diese Aufgabe ist dem Hauptanspruch zu entnehmen. Vorteilhafte Anwendungen werden in den nebengeordneten Ansprüchen aufgezeigt. Vorteilhafte Modifikationen sind den Unteransprüchen zu entnehmen und werden im Folgenden im Zusammenhang mit der Erfindung näher erläutert.

Das erfindungsgemäße Messverfahren basiert auf der überraschenden Erkenntnis, dass Kernholz und Splintholz bei einer speziellen Anregungswellenlänge unterschiedliche Fluoreszenzeigenschaften aufweisen. Bei der Erfindung stellte sich durch eine Überlagerung der normierten Fluoreszenzspektren heraus, dass diese einen charakteristischen Schnittpunkt bei einer bestimmten Wellenlänge aufweisen. Somit sind die Intensitäten bei allen anderen Wellenlängen unterschiedlich groß. Dabei ist es von der untersuchten Holzsorte abhängig, ob die Intensität für Kernholz oder Splintholz bei Wellenlängen größer oder kleiner als die Wellenlänge des Schnittpunkts jeweils größer oder kleiner ist. Kernholz und Splintholz können also dadurch eindeutig identifiziert werden, in welcher Größenrelation die Fluoreszenzintensitäten eines an einem beliebigen Messort in zwei festgelegten Wellenlängen, die größer bzw. kleiner als die Wellenlänge des Schnittpunkts gewählt sind, zueinander stehen. Mit der Erfindung wird ein nicht-invasives d.h. zerstörungs- und beschädigungsfreies, Messverfahren zur Verfügung gestellt, mit dem in äußerst einfacher und damit schneller, aber auch zuverlässiger Weise eine Unterscheidung zwischen Kernholz und Splintholz vorgenommen werden kann. Damit eignet sich das erfindungsgemäße Messverfahren speziell besonders zur automatisierten in-situ Untersuchung von Holz in der Massenproduktion.

Das erfindungsgemäße Messverfahren gliedert sich prinzipiell in eine Testphase und eine Messphase. Die Testphase umfasst erfindungsgemäß zumindest folgende Verfahrensschritte:
• Bestrahlen einer massiven Holzprobe einer bekannten Holzart mit monochromatischem Licht einer Fluoreszenz anregenden Wellenlänge λₐ zumindest an einem Messort M₁ in einem angenommenen Gebiet von Kernholz und zumindest an einem Messort M₂ in einem angenommenen Gebiet von Splintholz,
• Detektieren eines Fluoreszenzspektrums F₁ am Messort M₁ und eines Fluoreszenzspektrums F₂ am Messort M₂,
• Normieren, Überlagern und Analysieren der Fluoreszenzspektren F₁, F₂ hinsichtlich eines Schnittpunkts S mit dem Analyseergebnis:
   • kein Schnittpunkt S tritt auf, dann
      • Wiederholen der Testphase mit einer anderen Fluoreszenz anregenden Wellenlänge λₐ und/oder an anderen Messorten Mᵢ und/oder Beenden der Messverfahrens
      oder
   • ein Schnittpunkt S tritt bei einer Wellenlänge λ_{S} auf, dann
      • Detektieren von Intensitäten I₁(λ₁), I₁(λ₂) und I₂(λ₁), I₂(λ₂) in den Fluoreszenzspektren F₁, F₂ bei Wellenlängen λ₁<λ_{S} und λ₂>λ_{S},
      • Feststellen der Größenrelation der Intensitäten I₁(λ₁), I₁(λ₂) mit einer Zuordnung zu Kernholz (KH) und der Größenrelation der Intensitäten I₂(λ₁), I₂(λ₂) mit einer Zuordnung zu Splintholz (SH)

Die Testphase dient der Beurteilung einer Holzprobe einer beliebigen, aber bekannten Holzart für ihre Eignung in der Messphase. Dazu wird zunächst ermittelt, ob die Holzprobe eine auswertbare Unterscheidung von Kernholz und Splintholz aufweist. Die Oberfläche der Holzprobe wird mit unterschiedlichen, Fluoreszenz anregenden Wellenlängen bestrahlt. Dabei werden gezielt nur Messorte ausgewählt, an denen Kernholz bzw. Splintholz angenommen wird. Die detektierten Fluoreszenzspektren werden normiert und überlagert und auf einen Schnittpunkt S hin untersucht. Wird kein Schnittpunkt ermittelt, werden Messorte und Anregungswellenlänge verändert und weiter nach einem Schnittpunkt gesucht, oder es wird entschieden, dass die untersuchte Holzart keine charakteristische Unterscheidung von Kernholz und Splintholz aufweist und damit für die eigentliche Messphase nicht geeignet ist.

Bei einer erfolgreichen Testphase mit Auftreten eines Schnittpunkts der beiden normierten Fluoreszenzspektren für Kernholz und Splintholz bei einer bestimmten anregenden Wellenlänge kann dann Holz von der Art der Holzprobe in der Messphase automatisch auf Kernholz oder Splintholz geprüft werden. Die Messphase umfasst erfindungsgemäß zumindest folgende Verfahrensschritte:
• Bestrahlen eines massiven Holzprüflings der bekannten Holzart an zumindest einem beliebigen Messort Mᵢ mit der in der Testphase ermittelten Fluoreszenz anregenden Wellenlänge λₐ,
• Detektieren von Intensitäten Iᵢ(λ₁), Iᵢ(λ₂) bei den in der Testphase festgelegten Wellenlängen λ₁, λ₂,
• Feststellen der Größenrelation der Intensitäten Iᵢ(λ₁), Iᵢ(λ₂) und Zuordnen des Messorts Mᵢ zu Kernholz oder Splintholz anhand der in der Testphase ermittelten Größenrelationen und
• Beenden oder Wiederholen der Messphase an weiteren Messorten Mᵢ und/oder weiteren Holzprüflingen.

In der Messphase müssen an einem Messort Mᵢ nur zwei Intensitätswerte bei vorgegebenen Wellenlängen größer und kleiner als die Wellenlänge des Schnittpunkts miteinander verglichen und in eine Größenrelation zueinander gesetzt werden. Durch Vergleich mit der ermittelten Größenrelation aus der Testphase kann sofort als Ergebnis ausgegeben werden, ob der Messort Mᵢ auf dem aktuellen Holzprüfling in einem Gebiet mit Kernholz oder Splintholz liegt.

Das erfindungsgemäße Messverfahren gliedert sich prinzipiell in eine Testphase und eine Messphase. Diese können aber auch völlig unabhängig voneinander, insbesondere an unterschiedlichen Orten, zu völlig unterschiedlichen Zeiten und von unterschiedlichen Anwendern, durchgeführt werden. Beispielsweise kann die Testphase von einem Institut für Holztechnik oder von einer Lasertechnikfirma an einem ersten Ort und die Messphase von einem Sägewerk oder einem Möbelbauer an einem anderen Ort durchgeführt werden. Dabei muss die Testphase pro Holzart nur einmal durchgeführt werden, um die relevanten Parameterwerte zu ermitteln. Die Parameterwerte können dann entsprechend Dritten, insbesondere Anwendern, zur Verfügung gestellt werden. Besonders vorteilhaft kann daher ausschließlich die Messphase für eine bekannte Holzart durchgeführt werden, wenn die zu der bekannten Holzart zugehörigen Parameterwerte für die Fluoreszenz anregende Wellenlänge λₐ, die Wellenlängen λ₁, λ₂ und die Zuordnung der Größenrelation der Intensitäten Iᵢ(λ₁), Iᵢ(λ₂) zu Kernholz oder Splintholz aus der Testphase bekannt sind.

Besonders bevorzugt können die ermittelten Größenrelationen durch Quotientenbildung in Zahlenwerte Zᵢ überführt werden. Dabei ergibt sich dann für Kernholz und Splintholz je nach Holzart jeweils ein Zahlenwert größer oder kleiner 1. Bei der Messung von aktuellen Holzprüflingen muss dann nur noch der Zahlenwert durch Quotientenbildung aus den detektierten Intensitäten ermittelt und mit den bekannten Zahlenwerten für Kernholz und Splintholz verglichen werden. Die Intensitäten werden bei vorgegebenen Wellenlängen λ₁, λ₂ detektiert. Vorteilhaft ist dabei eine Abweichung von ± 5...10 nm zulässig, ohne dass sich an der zu ermittelnden Größenrelation etwas ändert. In der Regel haben die normierten Fluoreszenzspektren keinen weiteren Schnittpunkt über der Wellenlänge λ_{S} mehr. Die Wellenlänge λ₁ kann daher jede Wellenlänge im Fluoreszenzspektrum annehmen, die kleiner ist als die Wellenlänge λ_{S} des Schnittspunkts. Ebenso kann die Wellenlänge λ₂ jede Wellenlänge im Fluoreszenzspektrum annehmen, die größer ist als die Wellenlänge λ_{S} des Schnittpunkts ist. Da aber in der Nähe des Schnittpunkts S bei der Wellenlänge λ_{S} die größten Unterschiede zwischen den Fluoreszenzspektren auftreten, ist es vorteilhaft, die Wellenlängen λ₁, λ₂ in der Nähe von der Wellenlänge λₛ zu wählen, beispielsweise besonders bevorzugt die Wellenlänge λ₁ in einem Bereich von 100 nm bis 250 nm < λₛ und die Wellenlänge λ₂ in einem Bereich von 100 nm bis 250 nm > λₛ. Darüber hinaus kann bei einer Wahl möglichst nahe an der Wellenlänge λ_{S} sichergestellt werden, dass bei ggfs. doch auftretenden weiteren Schnittpunkten der beiden Fluoreszenzspektren diese unbeachtlich bleiben. Gleiches gilt für mögliche Artefakte in den Fluoreszenzspektren, die aus physikalischen Gründen auftreten (z.B. zweite Oberschwingung).

Sowohl in der Test- als auch in der Messphase können die Bereiche von Kernholz und Splintholz besonders einfach aufgefunden und auch gegeneinander abgegrenzt werden (Auffinden der Grenze zwischen Kernholz und Splintholz), wenn bevorzugt vorteilhaft die Messorte Mᵢ entlang einer die Kern- und Splintholzbereiche durchkreuzenden Scanlinie auf dem Holzprüfling angeordnet sind. Dabei kann vorteilhaft die Scanlinie entlang der Breite eines brettförmig ausgebildeten Holzprüflings verlaufen. Bei einer industriellen Produktion können dann beispielsweise Holzbretter über ihre Breite gescannt werden, um die beidseitigen Grenzen zwischen Kernholz und Splintholz aufzufinden. Entsprechend kann das Splintholz dann beispielsweise entfernt werden.

Bereits weiter oben wurde ausgeführt, dass das Auffinden der richtigen anregenden Wellenlänge für den Verlauf der Fluoreszenzspektren ausschlaggebend ist. Weist die Holzprobe eine charakteristische Unterscheidung von Kernholz und Splintholz auf, ist es immer möglich, durch Einstellen der speziellen Anregungswellenlänge zwei Fluoreszenzspektren zu erhalten, die in normierter Form den geforderten Schnittpunkt und die Unterscheidbarkeit der beiden Fluoreszenzspektren beiderseits des Schnittpunkts aufweisen, sodass das Messverfahren nach der Erfindung auch in der Messphase angewendet werden kann. Bevorzugt kann die anregende Wellenlänge in einem Bereich zwischen 250 nm und 550 nm liegen. Insbesondere wurden geeignete Fluoreszenzspektren bei einer Anregung mit monochromatischem Licht mit λ_{A}= 260 nm, 337 nm, 450 nm, 480 nm oder 520 nm aufgefunden. Bevorzugt können dann zur Detektion der speziellen, aussagekräftigen Intensitäten Wellenlängen λ₁ (im Bereich < λ_{S}) zwischen 350 nm und 550 nm, insbesondere 400 nm, 505 nm oder 520 nm, und Wellenlängen λ₂ (im Bereich im Bereich > λ_{S}) zwischen 450 nm und 650 nm, insbesondere 500 nm, 605 nm oder 620 nm, ausgewählt werden. Bei der Fluoreszenz anregenden Wellenlänge λₐ kann eine Abweichung von ± 2...5 nm zugelassen werden.

Zu den Holzarten mit einer Unterscheidbarkeit von Kern- und Splintholz zählen Kiefer, Tanne, Lärche, Eibe, Douglasie, Eiche, Buche, Rotbuche, Robinie, Ulme, Esche, Kirsche, Nuss, Ebenholz, Teak, Mahagoni oder Palisander. deren Holz ist somit für das Messverfahren nach der Erfindung besonders geeignet. Grundsätzlich können aber alle Holzarten in der Testphase des Messverfahrens auf eine Unterscheidbarkeit von Kernholz und Splintholz hin untersucht werden. Solche Holzarten, die sich in der Testphase als geeignet erweisen, weil sie über Kernholz und Splintholz verfügen, können dann in der Messphase bei der kommerziellen Verarbeitung der entsprechenden Holzart eingesetzt werden.

Zu den Holzarten, die eine besonders ausgeprägte Unterscheidung zwischen (echtem) Kernholz und Splintholz aufweisen, gehören Kiefernholz und Eichenholz (alle Sorten). Somit kann das Messverfahren nach der Erfindung besonders vorteilhaft zur Detektion von Kernholz und Splintholz bei diesen Holzarten angewendet werden. Bei einer Holzprobe und einem Holzprüfling aus Kiefernholz beträgt bevorzugt die anregende Wellenlänge λₐ 337 nm, die Wellenlänge λ₁ 400 nm und die Wellenlänge λ₂ 500 nm. Dabei ist für das Kernholz des Kiefernholzes die detektierte Intensität Iᵢ (λ₁) bei der Wellenlänge λ₁ größer als die detektierte Intensität Iᵢ (λ₂) bei der Wellenlänge λ₂ und für das Splintholz des Kiefernholzes die detektierte Intensität Iᵢ (λ₁) bei der Wellenlänge λ₁ kleiner als die detektierte Intensität Iᵢ (λ₂) bei der Wellenlänge λ₂. Im Messverfahren zeigt also eine größere Intensität bei der Wellenlänge λ₁ als bei der Wellenlänge λ₂ an, dass sich der Messort Mᵢ in einem Kernholzgebiet befindet, wohingegen ein Splintholzgebiet gemessen wurde, wenn die Intensität bei der Wellenlänge λ₁ kleiner ist als bei der Wellenlänge λ₂. Bevorzugt kann eine einfache Quotientenbildung zur Ermittlung von Zahlenwerten Z vorgenommen werden. Dabei beträgt bevorzugt bei Kiefernholz der Quotient Z₁ für Kernholz 1,2 ± 0,2 und der Quotient Z₂ für Splintholz 0,6 ± 0,1.

Bei einer Holzprobe und einem Holzprüfling aus Eichenholz beträgt bevorzugt die anregende Wellenlänge λₐ 450 nm, die Wellenlänge λ₁ 505 nm und die Wellenlänge λ₂ 605 nm. Dabei ist für das Kernholz des Eichenholzes die detektierte Intensität Iᵢ (λ₁) bei der Wellenlänge λ₁ kleiner als die detektierte Intensität Iᵢ(λ₂) bei der Wellenlänge λ₂ und für das Splintholz des Eichenholzes die detektierte Intensität Iᵢ (λ₁) bei der Wellenlänge λ₁ größer als die detektierte Intensität Iᵢ (λ₂) bei der Wellenlänge λ₂. Im Messverfahren zeigt also eine kleinere Intensität bei der Wellenlänge λ₁ als bei der Wellenlänge λ₂ an, dass sich der Messort Mᵢ in einem Kernholzgebiet befindet, wohingegen ein Splintholzgebiet gemessen wurde, wenn die Intensität bei der Wellenlänge λ₁ größer ist als bei der Wellenlänge λ₂. Bevorzugt kann wieder eine einfache Quotientenbildung zur Ermittlung von Zahlenwerten vorgenommen werden. Dabei beträgt bevorzugt bei Eichenholz der Zahlenwert Z₁ für Kernholz 0,90 ± 0,15 und der Zahlenwert Z₂ für Splintholz 1,2 ± 0,2.

Viele der für eine Anwendung der Messphase geeigneten Holzarten, insbesondere aber auch die heimischen Holzarten Kiefernholz und Eichenholz werden besonders intensiv in der holzverarbeitenden Industrie genutzt. Besonders vorteilhaft ist daher eine Anwendung des Messverfahrens nach der Erfindung zur Unterscheidung von Kernholz und Splintholz auf Basis von monochromatisch angeregten Fluoreszenzspektren in der Messphase - da die erforderlichen Parameterwerte aus der Testphase bereits bekannt sind - für eine zerstörungsfreie, automatische in-situ Unterscheidung von Kernholz und Splintholz von bewegten Holzprüflingen in der holzindustriellen Verarbeitung. Die Testphase kann in der holzindustriellen Verarbeitung zusätzlich auch besonders vorteilhaft zur Kalibrierung der jeweiligen Messvorrichtung eingesetzt werden, um im eigentlichen Messbetrieb zuverlässig korrekte Messwerte zu erhalten. Es wird dabei eine bereits vermessene Holzprobe der bekannten Holzart mit bekannten Parameterwerten auf der Messvorrichtung vermessen und die detektierten Parameterwerte an die bekannten Parameterwerte angeglichen. Weiterhin kann beispielsweise bei einem Holzinstitut, auch ausschließlich die Testphase des Verfahrens nach der Erfindung angewendet werden, um eine Eignungsuntersuchung und Parameterermittlung einer massiven Holzprobe einer bekannten Holzart durchzuführen. Die bei einer festgestellten Eignung der untersuchten Holzart ermittelten Parameterwerte können dann an jeden Anwender der Messphase des Verfahrens nach der Erfindung, der die untersuchte Holzart verarbeitet, weitergegeben werden. Weitere Einzelheiten zu dem Messverfahren nach der Erfindung sind den nachfolgenden Ausführungsbeispielen zu entnehmen.

### Ausführungsbeispiele

Das nicht-invasive Messverfahren zur Unterscheidung von Kernholz und Splintholz auf Basis von monochromatisch angeregten Fluoreszenzspektren nach der Erfindung wird nachfolgend anhand der schematischen Figuren beispielhaft näher erläutert. Dabei zeigt:
- **Figur 1**: eine schematische Übersicht über das Messverfahren,
- **Figur 2**: ein Fluoreszenzspektrum für Kiefernholz und
- **Figur 3**: ein Fluoreszenzspektrum für Eichenholz.

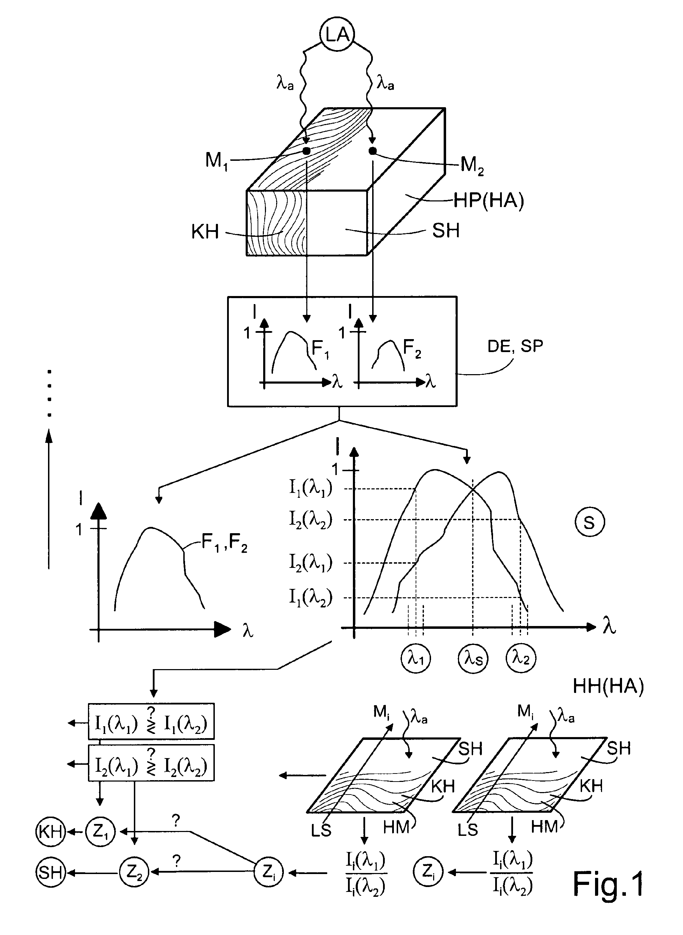

In der **Figur 1** wird eine schematische Übersicht über das Messverfahren nach der Erfindung mit Testphase und Messphase aufgezeigt.

In der Testphase (dargestellt bis zur gestrichelten Linie) wird eine massive Holzprobe **HP** einer bekannten Holzart **HA,** bei der das Vorhandensein von Kernholz **KH** und Splintholz **SH** angenommen wird, mit monochromatischem Licht einer Fluoreszenz anregenden Wellenlänge λₐ bestrahlt. Dabei kann zur Bestrahlung bevorzugt ein Laser **LA,** beispielsweise ein Stickstofflaser mit gepulstem Ausgangssignal, eingesetzt werden. Das Laserlicht der Wellenlänge λₐ wird an einem ersten Messort **M**₁ und an einem zweiten Messort **M**₂ eingestrahlt. Dabei ist der Messort **M**₁ so gewählt, dass er in einem angenommenen Gebiet von Kernholz **KH** liegt. Der Messort **M**₂ liegt hingegen in einem angenommenen Gebiet von Splintholz **SH.** Mit einem Detektor **DE,** beispielsweise ein einfacher photoelektrischer Empfänger in einem Spektroskop SP, das auch für die Auswertung genutzt wird, werden in den Messorten **M₁, M₂** dann Fluoreszenzspektren **F₁**, **F₂** (nur schematisch dargestellt) detektiert (Verlauf der Intensität I des emittierten fluoreszierenden Lichts in Abhängigkeit von dessen Wellenlänge λ) und normiert. Wenn die Fluoreszenzspektren **F₁**, **F₂** deutlich unterschiedlich sind, ist eine Normierung auf 1 mit einer direkten Proportionalität der beiden Fluoreszenzspektren **F₁**, **F₂** sinnvoll.

Anschließend werden die beiden Fluoreszenzspektren **F₁**, **F₂** überlagert und analysiert. Dabei wird nach einem Schnittpunkt **S** der beiden Fluoreszenzspektren **F₁**, **F₂** gesucht. Tritt kein Schnittpunkt **S** auf, wird die Testphase mit einer anderen Fluoreszenz anregenden Wellenlänge λₐ wiederholt. Eine weitere Variation ist die Veränderung des Messorts **Mᵢ.** Tritt bei keiner der beiden Variationen in irgendeiner Kombination von anregender Wellenlänge λₐ und Messort **Mᵢ** ein Schnittpunkt **S** der beiden Fluoreszenzspektren **F₁**, **F₂** auf, so weist die Holzprobe **HP** der gewählten Holzart **HA** keine Unterscheidung von Kernholz **KH** und Splintholz **SH** auf und ist für eine Anwendung des Messverfahrens nach der Erfindung zu einer Unterscheidung zwischen Kernholz **KH** und Splintholz **SH** nicht geeignet. Das Messverfahren wird dann beendet.

Ergibt sich jedoch bei einer Kombination von anregender Wellenlänge λₐ und Messort **Mᵢ** ein Schnittpunkt **S** bei einer Wellenlänge λ_{S} der beiden Fluoreszenzspektren **F₁**, **F₂**, weist die gewählte Holzart **HA** eine charakteristische Unterscheidung zwischen Kernholz **KH** und Splintholz **SH** auf und ist für die Messphase des Messverfahrens nach der Erfindung geeignet. Die Unterschiedlichkeit der Fluoreszenzspektren **F₁**, **F₂** bei Wellenlängen kleiner λₛ und bei Wellenlängen größer λₛ ermöglicht die weitere Auswertung mithilfe von Intensitätswerten Iᵢ der beiden Fluoreszenzspektren **F₁**, **F₂** bei verschiedenen ausgewählten Wellenlängen λ.

In der Testphase wird anschließend eine Wellenlänge λ₁ < λ_{S} bestimmt, bei der Intensitäten I₁(λ₁), I₂(λ₁) detektiert werden. Dabei wird die Wellenlänge λ₁ in einem Bereich von 100 nm bis 250 nm < λ_{S} ausgewählt. Analog wird eine Wellenlänge λ₂ > λ_{S} festgelegt, bei der Intensitäten I₁(λ₂), I₂(λ₂) detektiert werden. Dabei wird die Wellenlänge λ₂ in einem Bereich von 100 nm bis 250 nm > λ_{S} ausgewählt. Eine Abeichung der Wellenlängen λ₁, λ₂ von ± 5 nm bis 10 nm ist problemlos zulässig. Anschließend werden die Intensitäten I₁(λ₁), I₁(λ₂) im Fluoreszenzspektrum **F₁** und die Intensitäten I₂(λ₁), I₂(λ₂) im Fluoreszenzspektrum **F₂** detektiert. Im nächsten Verfahrensschritt wird dann die Größenrelation der Intensitäten I₁(λ₁), I₁(λ₂) im Fluoreszenzspektrum **F₁** festgestellt. Es wird festgestellt, ob I₁(λ₁) größer oder kleiner als I₁(λ₂) ist. Da das Fluoreszenzspektrum **F₁** am Messort **M₁** im Gebiet von Kernholz **KH** detektiert wurde, kann die ermittelte Größenrelation eindeutig dem Kernholz **KH** zugeordnet werden. Analog wird festgestellt, ob I₂(λ₁) größer oder kleiner als I₂(λ₂) ist. Hierbei ist es zwangsläufig, dass sich aufgrund des Schnittpunkts **S** der beiden Fluoreszenzspektren **F₁**, **F₂** nunmehr die andere Größenrelation ergibt. Da das Fluoreszenzspektrum **F₂** am Messort **M₂** im Gebiet von Splintholz **SH** detektiert wurde, kann die ermittelte Größenrelation eindeutig dem Splintholz **SH** zugeordnet werden.

Nunmehr kann in der Messphase für jeden Holzprüfling **HH,** beispielsweise ein Brett, einer durch die Testphase in ihren Parameterwerten bekannten Holzart **HA** durch Detektieren der Intensitäten I₁(λ₁), I₂(λ₂), I₂(λ₁), I₂(λ₂) bei den vorbestimmten Wellenlängen λ₁, λ₂ ± 5 nm bis 10 nm und Feststellung der Größenrelation unmittelbar eine schnelle und zuverlässige Zuordnung des jeweiligen Messorts **Mᵢ** zu Kernholz **KH** oder Splintholz **SH** erfolgen. Dabei kann die Zuordnung noch vereinfacht werden, wenn aus den ermittelten Größenrelationen der beiden Fluoreszenzspektren **F₁**, **F₂** für Kernholz **KH** und Splintholz **SH** Zahlenwerte **Z₁**, **Z₂** als Quotienten gebildet werden, die entsprechend größer oder kleiner 1 sind. In der Messphase wird dann für jeden Messort **Mᵢ** ein Zahlenwert **Zᵢ** als Quotient aus den entsprechenden Intensitäten I₁(λ₁)/ I₁(λ₂) bzw. I₂(λ₁)/ I₂(λ₂) gebildet und mit **Z₁**, **Z₂** aus der Testphase verglichen, um die schnelle und zuverlässige Zuordnung zu Kernholz **KH** oder Splintholz **SH** zu erhalten.

Die bei Kenntnis der Parameterwerte für eine Holzart **HA** aus der Testphase völlig eigenständig durchführbare Messphase beginnt mit dem Bestrahlen eines massiven Holzprüflings **HH** der bekannten Holzart **HA** zumindest an einem beliebigen Messort **Mᵢ** mit der in der Testphase festgelegten, Fluoreszenz anregenden Wellenlänge λₐ (Abweichung ± 2...5 nm). Dabei liegen die Messorte Mᵢ bevorzugt auf einer Linie **LS** (Linienscan), wobei sich die Linie **LS** quer zur Holzmaserung **HM** erstreckt. Anschließend werden die Intensitäten Iᵢ(λ₁), Iᵢ(λ₂) bei den Wellenlängen λ₁, λ₂ detektiert und die Größenrelationen der Intensitäten Iᵢ(λ₁), Iᵢ(λ₂) bzw. die Zᵢ der Quotienten bestimmt. An dieser Stelle sei bemerkt, dass ein Anwender natürlich auch andere geeignete Wellenlängen λ₁, λ₂ festlegen kann, wenn er die Fluoreszenzspektren **F₁**, **F₂** und die einzuhalten Bedingungen kennt. In Analogie zu den festgestellten Größenrelationen bzw. Zahlenwerten **Z₁**, **Z₂** der Quotienten in der Testphase erfolgt dann die Zuordnung des Messorts **Mᵢ** zu Kernholz **KH** oder Splintholz **SH.** Die Messphase wird bevorzugt für eine nicht-invasive (zerstörungs- und beschädigungsfreie), automatische in-situ Unterscheidung von Kernholz **KH** und Splintholz **SH** von bewegten Holzprüflingen **HH** in der holzindustriellen Verarbeitung bei Zuschnitt- und Sortierprozessen oder bei anderen Bearbeitungsprozessen, wie beispielsweise Beizen, Lackieren, Beschichten, Hobeln oder Schleifen, angewendet.

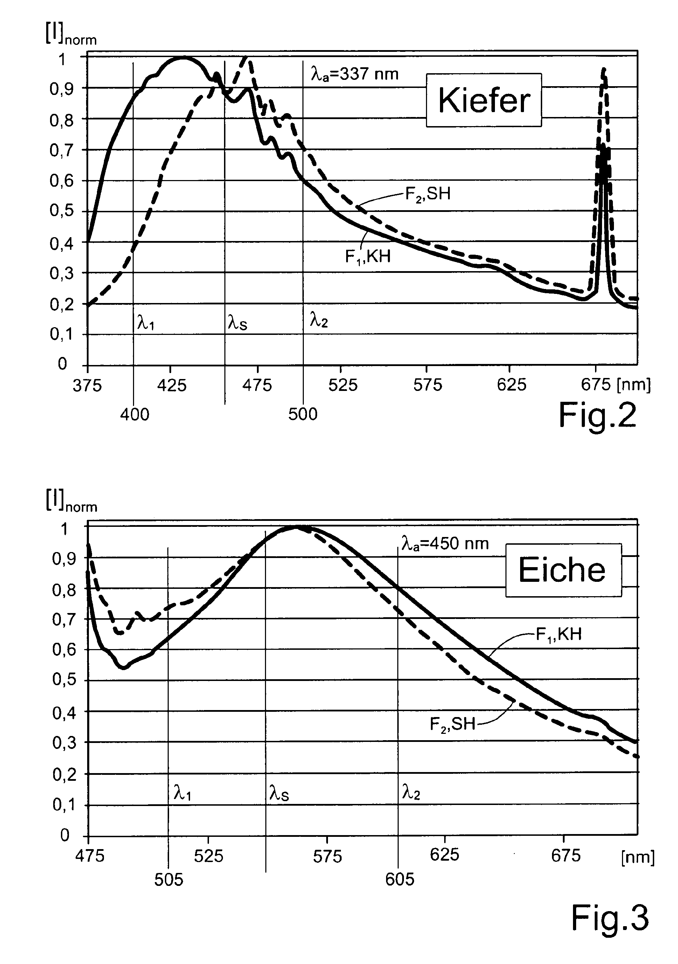

Die **Figur 2** zeigt für Kiefernholz bei einer anregenden Wellenlänge λₐ das normierte Fluoreszenzspektrum **F₁** für Kernholz **KH** und das auf 1 normierte Fluoreszenzspektrum **F₂** für Splintholz **SH.** Deutlich ist deren Schnittpunkt **S** zu erkennen. Ebenfalls eingezeichnet sind die ausgewählten Wellenlängen λ₁, λ₂. Bei Kiefernholz ist für Kernholz **KH** der Zahlenwert Z₁ > 1 und für Splintholz **SH** ist der Zahlenwert Z₂ < 1. Alle zugehörigen Parameterwerte sind der nachfolgenden **Tabelle 1** zu entnehmen. Bei dem Peak am rechten Rand des Diagramms handelt es sich um ein Artefakt. Die Wellenlänge λ₂ ist entsprechend unterhalb des Artefakts zu wählen.

| **Tabelle 1, Kiefernholz** | **Kernholz** | **Splintholz** |
|---|---|---|
| Fluoreszenzspektrum | F₁ | F₂ |
| Anregung bei Wellenlänge λₐ | 337 nm ± 2...5 nm | 337 nm ± 2...5 nm |
| Detektion bei Wellenlänge λ₁ | 400 nm ± 5...10 nm | 400 nm ± 5...10 nm |
| Detektion bei Wellenlänge λ₂ | 500 nm ± 5...10 nm | 500 nm ± 5...10 nm |
| Größenrelation | I₁(λ₁) > I₁(λ₁) | I₂(λ₁) < I₂(λ₁) |
| Zahlenwert Zᵢ | Z₁ = 1,2 ± 0,2 | Z₂ = 0,6 ± 0,1 |

Die **Figur 3** zeigt für Eichenholz bei einer anregenden Wellenlänge λₐ das normierte Fluoreszenzspektrum **F₁** für Kernholz **KH** und das normierte Fluoreszenzspektrum **F₂** für Splintholz **SH.** Deutlich ist deren Schnittpunkt **S** zu erkennen. Ebenfalls eingezeichnet sind die ausgewählten Wellenlängen λ₁, λ₂. Bei Eichenholz ist für Kernholz **KH** der Zahlenwert Z₁ < 1 und für Splintholz **SH** ist der Zahlenwert Z₂ > 1. Alle zugehörigen Parameterwerte sind der nachfolgenden **Tabelle 2** zu entnehmen.

| **Tabelle 2, Eichenholz** | **Kernholz** | **Splintholz** |
|---|---|---|
| Fluoreszenzspektrum | F₁ | F₂ |
| Anregung bei Wellenlänge λₐ | 450 nm ± 2...5 nm | 450 nm ± 2...5 nm |
| Detektion bei Wellenlänge λ₁ | 505 nm ± 5...10 nm | 505 nm ± 5...10 nm |
| Detektion bei Wellenlänge λ₂ | 605 nm ± 5...10 nm | 605 nm ±5...10 nm |
| Größenrelation | I₁(λ₁) < I₁(λ₁) | I₂(λ₁) > I₂(λ₁) |
| Zahlenwert Zᵢ | Z₁ = 0,90 ± 0,15 | Z₂= 1,2 ± 0,2 |

### Bezugszeichenliste

- **DE**: Detektor
- **F**: Fluoreszenzspektrum
- **HA**: Holzart
- **HH**: Holzprüfling (Messphase)
- **HM**: Holzmaserung
- **HP**: Holzprobe (Testphase)
- **I**: Intensität
- **i**: Laufindex
- **KH**: Kernholz
- λ: Wellenlänge
- **LA**: Laser
- **LS**: Linienscan
- **M**: Messort
- **S**: Schnittpunkt
- **SH**: Splintholz
- **SP**: Spektroskop
- **Z**: Zahlenwert

## Patentansprüche

1. Nicht-invasives Messverfahren zur Unterscheidung von Kernholz (KH) und Splintholz (SH) auf Basis von monochromatisch angeregten Fluoreszenzspektren Fᵢ, mit einer
Testphase zumindest mit den Verfahrensschritten :
• Bestrahlen einer massiven Holzprobe (HP) einer bekannten Holzart (HA) mit monochromatischem Licht einer Fluoreszenz anregenden Wellenlänge λₐ zumindest an einem Messort M₁ in einem angenommenen Gebiet von Kernholz (KH) und zumindest an einem Messort M₂ in einem angenommenen Gebiet von Splintholz (SH),
• Detektieren eines Fluoreszenzspektrums F₁ am Messort M₁ und eines Fluoreszenzspektrums F₂ am Messort M₂,
• Normieren, Überlagern und Analysieren der Fluoreszenzspektren F₁, F₂ hinsichtlich eines Schnittpunkts S mit dem Analyseergebnis:
• kein Schnittpunkt S tritt auf, dann
• Wiederholen der Testphase mit einer anderen Fluoreszenz anregenden Wellenlänge λₐ und/oder an anderen Messorten Mᵢ und/oder Beenden der Messverfahrens
oder
• ein Schnittpunkt S tritt bei einer Wellenlänge λ_{S} auf, dann
• Detektieren von Intensitäten I₁(λ₁), I₁(λ₂) und I₂(λ₁), I₂(λ₂) in den Fluoreszenzspektren F₁, F₂ bei Wellenlängen λ₁<λ_{S} und λ₂>λ_{S},
• Feststellen der Größenrelation der Intensitäten I₁(λ₁), I₁(λ₂) mit einer Zuordnung zu Kernholz (KH) und der Größenrelation der Intensitäten I₂(λ₁), I₂(λ₂) mit einer Zuordnung zu Splintholz (SH)
und mit einer
Messphase zumindest mit den Verfahrensschritten:
• Bestrahlen eines massiven Holzprüflings (HH) der bekannten Holzart (HA) an zumindest einem beliebigen Messort Mᵢ mit der in der Testphase ermittelten Fluoreszenz anregenden Wellenlänge λₐ,
• Detektieren von Intensitäten Iᵢ(λ₁), Iᵢ(λ₂) bei den in der Testphase festgelegten Wellenlängen λ₁, λ₂,
• Feststellen der Größenrelation der Intensitäten Iᵢ(λ₁), Iᵢ(λ₂) und Zuordnen des Messorts Mᵢ zu Kernholz (KH) oder Splintholz (SH) anhand der in der Testphase ermittelten Größenrelationen und
• Beenden oder Wiederholen der Messphase an weiteren Messorten Mᵢ und/oder weiteren Holzprüflingen (HH).

2. Messverfahren nach Anspruch 1,
**dadurch gekennzeichnet, dass**
ausschließlich die Messphase für eine bekannte Holzart (HA) durchgeführt wird, wenn die zu der bekannten Holzart (HA) zugehörigen Parameterwerte für die Fluoreszenz anregende Wellenlänge λₐ, die Wellenlängen λ₁, λ₂ und die Zuordnung der Größenrelation der Intensitäten Iᵢ(λ₁), Iᵢ(λ₂) zu Kernholz (KH) oder Splintholz (SH) aus der Testphase bekannt sind.

3. Messverfahren nach Anspruch 1 oder 2,
**dadurch gekennzeichnet, dass**
die Größenrelationen durch Quotientenbildung in Zahlenwerte Zᵢ überführt werden.

4. Messverfahren nach zumindest einem der Ansprüche 1 bis 3,
**dadurch gekennzeichnet, dass**
für die Fluoreszenz anregenden Wellenlänge λₐ eine Abweichung von ± 2...5 nm, für die Wellenlängen λ₁, λ₂ eine Abweichung von ± 5...10 nm zulässig und/oder dass die Wellenlänge λ₁ in einem Bereich von 100 nm bis 250 nm < λₛ und die Wellenlänge λ₂ in einem Bereich von 100 nm bis 250 nm > λₛ ausgewählt ist.

5. Messverfahren nach zumindest einem der Ansprüche 1 bis 4,
**dadurch gekennzeichnet, dass**
die Messorte Mᵢ entlang einer Scanlinie (SL) auf der Holzprobe (HP) und/oder dem Holzprüfling (HH) angeordnet sind.

6. Messverfahren nach Anspruch 5,
**dadurch gekennzeichnet, dass**
die Scanlinie quer zur Holzmaserung (HM) einer brettförmig ausgebildeten Holzprobe (HP) und/oder Holzprüflings (HH) verläuft.

7. Messverfahren nach zumindest einem der Ansprüche 1 bis 6,
**dadurch gekennzeichnet, dass**
die Holzprobe (HP) und/oder der Holzprüfling (HH) aus dem Holz der Kiefer, Tanne, Lärche, Eibe, Douglasie, Eiche, Buche, Robinie, Ulme, Esche, Kirsche, Nuss, Ebenholz, Teak, Mahagoni oder Palisander als Holzart (HA) bestehen.

8. Messverfahren nach Anspruch 7,
**dadurch gekennzeichnet, dass**
bei Kiefernholz als Holzart (HA) die Anregungswellenlänge λ_{A} 337 nm, die Wellenlänge λ₁ 400 nm und die Wellenlänge λ₂ 500 nm beträgt, wobei für das Kernholz (KH) des Kiefernholzes die detektierte Intensität Iᵢ(λ₁) bei der Wellenlänge λ₁ größer ist als die detektierte Intensität Iᵢ(λ₂) bei der Wellenlänge λ₂ und für das Splintholz (SH) des Kiefernholzes die detektierte Intensität Iᵢ(λ₁) bei der Wellenlänge λ₁ kleiner ist als die detektierte Intensität Iᵢ(λ₂) bei der Wellenlänge λ₂.

9. Messverfahren nach Anspruch 3 und 8,
**dadurch gekennzeichnet, dass**
bei Kiefernholz der Zahlenwert Z₁ für Kernholz (KH) 1,2 ± 0,2 und der Zahlenwert Z₂ für Splintholz (SH) 0,6 ± 0,1 beträgt.

10. Messverfahren nach Anspruch 7,
**dadurch gekennzeichnet, dass**
bei einer Holzprobe (HP) und einem Holzprüfling (HH) aus Eichenholz als Holzart (HA) die anregende Wellenlänge λₐ 450 nm, die Wellenlänge λ₁ 505 nm und die Wellenlänge λ₂ 605 nm beträgt, wobei für das Kernholz (KH) des Eichenholzes die detektierte Intensität Iᵢ(λ₁) bei der Wellenlänge λ₁ kleiner ist als die detektierte Intensität Iᵢ(λ₂) bei der Wellenlänge λ₂ und für das Splintholz (SH) des Eichenholzes die detektierte Intensität Iᵢ(λ₁) bei der Wellenlänge λ₁ größer ist als die detektierte Intensität Iᵢ(λ₂) bei der Wellenlänge λ₂.

11. Messverfahren nach Anspruch 3 und 10,
**dadurch gekennzeichnet, dass**
bei Eichenholz der Zahlenwert Z₁ für Kernholz (KH) 0,90 ± 0,15 und der Zahlenwert Z₂ für Splintholz (SH) 1,2 ± 0,2 beträgt.

12. Anwendung des nicht-invasiven Messverfahrens zur Unterscheidung von Kernholz (KH) und Splintholz (SH) auf Basis von monochromatisch angeregten Fluoreszenzspektren Fᵢ nach einem der Ansprüche 1 bis 11 in der Messphase für eine zerstörungsfreie, automatische in-situ Unterscheidung von Kernholz (KH) und Splintholz (SH) von bewegten Holzprüflingen (HH) in der holzindustriellen Verarbeitung.

13. Anwendung nach Anspruch 12 zusätzlich in der Testphase für eine Kalibrierung einer Messvorrichtung zur Durchführung der Messphase.

14. Anwendung des nicht-invasiven Messverfahrens zur Unterscheidung von Kernholz (KH) und Splintholz (SH) auf Basis von monochromatisch angeregten Fluoreszenzspektren Fᵢ nach zumindest einem der Ansprüche 1 bis 11 ausschließlich in der Testphase für eine Eignungsuntersuchung und Parameterermittlung einer massiven Holzprobe (HP) einer bekannten Holzart (HA) für die Messphase.
